# EUROPEAN PATENT SPECIFICATION

(11) **EP 2 721 037 B1**
(45) Date of publication and mention of the grant of the patent: **28.11.2018**
(21) Application number: 12731837.6
(22) Date of filing: 18.06.2012
(51) Int. Cl.: C07D 471/04, C07D 471/08, H01M 12/06

(54) **SYNTHESIS OF HETERO IONIC COMPOUNDS USING DIALKYLCARBONATE QUATERNIZATION**
SYNTHESE VON HETEROIONISCHEN VERBINDUNGEN UNTER VERWENDUNG VON DIALKYLCARBONATQUATERNIERUNG
SYNTHÈSE DE COMPOSÉS HÉTÉRO-IONIQUES À L'AIDE DE QUATERNARISATION DE DIALKYLCARBONATES

(30) Priority: 17.06.2011 US 201161498308 P; 09.12.2011 US 201161569159 P
(43) Date of publication of application: 23.04.2014
(60) Divisional application: 18155977.4
(73) Proprietor: NantEnergy, Inc., Scottsdale, AZ 85258 (US)
(72) Inventor: FRIESEN, Cody A., Fort McDowell, Arizona 85264 (US); WOLFE, Derek, Scottsdale, Arizona 85258 (US); JOHNSON, Paul, Bryan, Phoenix, Arizona 85032 (US)
(74) Representative: Beck Greener
(86) International application number: PCT/US2012/043013
(87) International publication number: WO 2012/174564

(56) References cited:
- EP-A1- 1 398 318
- WO-A2-2006/072785
- US-A1- 2008 251 759
- US-A1- 2010 285 375
- US-A1- 2011 027 664
- A. R. Hajipour ET AL: "Basic ionic liquids. A short review", IRANIAN CHEMICAL SOCIETY. JOURNAL, vol. 6, no. 4, 1 December 2009 (2009-12-01), pages 647-678, XP055323295, IR ISSN: 1735-207X, DOI: 10.1007/BF03246155

## Description

### FIELD OF THE INVENTION

The embodiments relate to methods of making a sulfate salt of 1,4,diazabicyclo[2,2,2] octane using a dialkylcarbonate as a primary reactant. Embodiments also relate to methods of making ionic liquids by reacting the sulfate salt of 1,4,diazabicyclo[2,2,2] octane with a salt to produce an ionic liquid comprising 1,4,diazabicyclo[2,2,2] octane and the anion of the salt, or by anion exchange to produce an ionic liquid comprising the heteroatomic cation and the anion. Embodiments also relate to use of the salt made as an ionic liquid and as an additive to an alkaline electrochemical cell.

### BACKGROUND OF THE INVENTION

A significant detriment to the energy density of most batteries is posed by the battery's cathode. This is true for battery chemistries using, for example, lithium or nickel. Typically, oxidant is stored at the cathode at a molar charge capacity that is two to five times lower than that of the anode. Many fuel cells, on the other hand, use oxygen from the air as a source of oxidant. The existence of a continuous and virtually limitless oxidant source enables, in principle, high energy density. However, the use of hydrogen and organic fuels precludes high energy efficiencies due to problems with vapor pressure and balance-of-systems complexity, such as humidification and membrane issues. Metal-air electrochemical cells are able to combine the ultra-high anode capacity of batteries with the air-breathing cathode of fuel cells in order to achieve substantial energy densities that are relevant to modern energy demands.

Metal-air batteries typically include a fuel electrode at which metal fuel is oxidized, an air electrode at which oxygen is reduced, and an electrolyte solution for providing ion conductivity. A limiting factor with metal-air batteries is the evaporation of the electrolyte solution, particularly the evaporation of the bulk solvent, such as water in an aqueous electrolyte solution. Because the air electrode is required to be air permeable to absorb oxygen, it is also may permit the solvent vapor, such as water vapor, to escape from the cell. Over time, the cell becomes incapable of operating effectively because of this issue. Indeed, in many cell designs this evaporation issue renders the cell inoperable before the fuel is consumed. And this issue is exacerbated in secondary (i.e., rechargeable) cells, because the fuel may be re-charged repeatedly over the life of the cell, whereas the electrolyte solution is not (absent replenishment from an external source). Also, in rechargeable cells the water solvent is typically oxidized to evolve oxygen during re-charge, which may also deplete the solution.

There are other problems associated with conventional aqueous electrolyte batteries, such as water electrolysis during recharging, and self discharge. During recharge, a current is passed through the battery to reduce the oxidized fuel at the fuel electrode. Some of the current, however, electrolyzes the water resulting in hydrogen evolution (reduction) at the fuel electrode and oxygen evolution (oxidation) at the oxygen electrode as represented in the following equations:

(1) Reduction: 2 H₂O(*l*) + 2e⁻ → H₂(*g*) + 2 OH⁻(*aq*)

and

(2) Oxidation: 2 H₂O(*l*) → O₂(*g*) + 4 H⁺(*aq*) + 4e⁻

In this manner, further aqueous electrolyte is lost from the battery. Additionally, the electrons that are consumed in reducing hydrogen are not available to reduce the fuel oxide. Therefore, the parasitic electrolysis of the aqueous electrolyte reduces the round trip efficiency of the secondary battery.

Self-discharge may result from impurities in the electrodes or reaction with the electrolyte. Typically, self-discharge from impurities in the electrodes is small (2-3% loss per month). The reaction of an active metal with water and/or O₂ dissolved in the water, however, may be quite high (20-30% per month).

To compensate for these problems, metal-air batteries with aqueous electrolyte solutions are typically designed to contain a relatively high volume of electrolyte solution. Some cell designs even incorporate means for replenishing the electrolyte from an adjacent reservoir to maintain the electrolyte level. However, either approach adds significantly to both the overall size of the cell, as well as the weight of the cell, without enhancing the cell performance (except to ensure that there is a sufficient volume of electrolyte solution to offset evaporation of the water or other solvent over time). Specifically, the cell performance is generally determined by the fuel characteristics, the electrode characteristics, the electrolyte characteristics, and the amount of electrode surface area available for reactions to take place. But the volume of electrolyte solution in the cell generally does not have a significant beneficial effect on cell performance, and thus generally only detracts from cell performance in terms of volumetric and weight based ratios (power to volume or weight, and energy to volume or weight). Also, an excessive volume of electrolyte may create a higher amount of spacing between the electrodes, which may increase ionic resistance and detract from performance.

Another problem that arises with alkaline electrolyte cells is the formation of filaments or dendrites during the charging/discharging cycle. For example, during the charging of a rechargeable electrochemical cell, metal cations in the electrolyte are reduced at the electrode and are electrodeposited onto the electrode as the metal. Ideally, the electrodeposited metal is laid down as a smooth layer over the entire electrode surface, thereby preserving the electrode surface morphology from one discharge-charge cycle to the next. In practice, however, the metal tends to deposit preferentially at certain sites on the electrode. As a consequence, the morphology of the metal deposit is such that the electrode surface undergoes modification ranging from moderate roughening to formation of a coating of filaments or dendrites over the entire surface. After several cycles, the electrode can become covered by a dense mat of interwoven dendrites. This type of metal deposition is undesirable and also hazardous in electrochemical cells because the metal dendrites are often small enough to penetrate the microporous materials that are conventionally used to separate the anode from the cathode current collector. As a consequence, the dendrites can grow through the separator material and cause a short-circuit between the electrodes, resulting in cell failure and possible explosion. Dendrite growth around the edges of the separator material can also occur with similar results.

The use of non-aqueous systems for electrochemical cells has been suggested (*see, e.g.,* U.S. Patent No. 5,827,602). In non-aqueous systems, the aqueous electrolyte may be replaced with an ionic liquid. Ionic liquids which contain a strong Lewis acid such as AlCl₃, however, are known to liberate toxic gases when exposed to moisture.

The use of low or room temperature ionic liquid rather than an aqueous electrolyte in a metal-air electrochemical cell, as described in U.S. Provisional Application Serial Nos. 61/383,510, filed September 16, 2010; 61/355,081, filed June 15, 2010; 61/334,047, filed May 12, 2010; 61/329,278, filed April 29, 2010; 61/177,072, filed May 11, 2009, and 61/267,240, filed December 7, 2009, and described in U.S. Patent Application Nos.: 13/105,794, filed on May 11, 2011; 13/096,851, filed April 28, 2011; 13/085714, filed April 13, 2011; and 12/776,962, filed May 10, 2010. The use of a low or room temperature ionic liquid in the cell essentially eliminates the problems associated with evaporation of solvent from an electrolytic solution.

Room temperature ionic liquids have extremely low vapor pressures (some have vapor pressures that are essentially immeasurable under standard conditions) and thus experience little or no evaporation. Therefore, cells using low or room temperature ionic liquids as their ionically conductive media need not incorporate excessive volumes of solution in order to compensate for evaporation over time. Relatively small amounts of ionic liquid are sufficient to support the electrochemical reactions needed for cell operation, thereby reducing cell weight and volume and increasing power to volume/weight ratios. Also, other problems associated with solvents, such as hydrogen evolution in an aqueous solution, may be avoided. This development is not conceded to be prior art and merely is described for contextual purposes to facilitate an understanding of the further development described herein.

Quaternization of tertiary amines, especially imidazole compounds and linear amines, by reaction with dimethyl carbonate is known. Some describe the use of the carboxylates or carbonates produced by this reaction as useful ionic liquid intermediates. *See, e.g.,* Holbrey, et al., "1,3-Dimethylimidazolium-2-carboxylate: the unexpected synthesis of an ionic liquid precursor and carbine-CO2 adduct," Chem. Commun. (2003), 28-29; Smiglak, et al., "Ionic Liquids via reaction of the zwitterionic 1,3,-dimethylimidazolium-2-carboxylate with protic acids,..." Green Chem., Vol. 9, pp. 90-98 (2006); Bridges, et al., "An Intermediate for the Clean Synthesis of Ionic Liquids... ," Chem. Eur. J., 5207- 5212 (2007); Holbrey, et al., "Optimized microwave-assisted synthesis of methylcarbonate salts;... ," Green Chem., Vol. 12, pp 407-413 (2010); Yang, et al., "Dimethyl Carbonate Synthesis catalyzed by DABCO-derived basic ionic liquids via transesterification of ethylene carbonate with methanol," Tetrahedron Letters, 51, pp 2931-2934 (2010); and U.S. Patent Nos. 4,892,944, and 5,865,513.
EP1398318 discloses a method for preparing an ionic liquid: (1) reacting a imidazolium compound with dialkylcarbonate to produce an alkylcarbonate salt of the heteroatomic compound (D1, paragraphs 10-56, especially paragraphs 42-49; (2) acidifying the alkylcarbonate salt with a sulfate-containing acid to produce a bisulfate salt of the imidazolium (D1, paragraphs 25-26, 50-54); and (3) converting the bisulfate salt of the imidazolium to an ionic liquid comprising an imidazolium cation and an anion (D1, paragraphs 55-56).

### SUMMARY

One feature of an embodiment provides a method for preparing an ionic liquid comprising: (1) reacting 1,4, diazabicyclo [2,2,2] octane with dialkylcarbonate to produce an alkylcarbonate salt of 1,4, diazabicyclo [2,2,2] octane; (2) acidifying the alkylcarbonate salt with with a sulfate-containing acid to produce a sulfate salt of 1,4, diazabicyclo[2,2,2] octane; and (3) converting the sulfate salt of 1,4, diazabicyclo[2,2,2] octane to an ionic liquid comprising an alkyl-1,4,diazabicyclo[2,2,2] octane cation and an anion. An additional feature of the embodiments provides a method for converting the sulfate salt of the heteroatomic compound to an ionic liquid comprising an alkyl-heteroatomic cation and an anion.

Described herein is an electrochemical metal-air cell comprising a fuel electrode for oxidizing a metal fuel; an air electrode for absorbing and reducing gaseous oxygen; and an ionically conductive medium comprising a low temperature ionic liquid having a melting point at or below 150°C at 1 atm, wherein the ionic liquid comprises a cation of the heteroatomic compound and an anion prepared in accordance with the method described above. The ionic liquid is contained in a space between the fuel electrode and the air electrode for conducting ions for supporting the electrochemical reactions at the fuel and air electrodes.

Also described herein is an electrochemical cell comprising a cathode, an anode, an aqueous electrolyte and an additive that includes a heteroatomic compound cation and an anion (hetero ionic compound or complex).

For the purposes of this application, a low temperature ionic liquid is defined as an ionic liquid having a melting point at or below 150°C at 1 atm. These low temperature ionic liquids may also include the species known as room temperature ionic liquids, which are defined as ionic liquids having a melting point at or below 100°C at 1 atm. Ionic liquids are also referred to as liquid salts. By definition, an ionic liquid is composed primarily of anions and cations of the salt. While an ionic liquid itself may be a solvent with respect to one or more other soluble products present in the ionic liquid, such as an additive or reactant by-product created by operation of the cell, an ionic liquid does not require the use of a solvent to dissolve the salt, as the liquid itself is "self-dissolving," i.e., it is a liquid of the electrolyte salt anions and cations by its own nature, and the use of a separate solvent to dissolve the salt is not necessary. However, the ionic liquid may in some embodiments be provided in a solvent, for example, as an additive.

However, even though low temperature or room temperature ionic liquids are defined by their respective melting points at 1 atm., in some embodiments the cell may be operated in an environment with a different pressure, and thus the melting point may vary with the operating pressure. Thus, reference to a melting point at 1 atm. is used as a reference point to define these liquids, and does not imply or restrict its actual use conditions in operation.

Other objects, features, and advantages of the present invention will become apparent from the following detailed description, the accompanying drawings, and the appended claims.

### BRIEF DESCRIPTION OF THE DRAWINGS

Fig. 1 is an exploded view of a cell; and
Fig. 2 is a schematic of an exemplary alkaline cell in accordance with an embodiment.

### DETAILED DESCRIPTION

Ionic liquids generally refer to salts that form stable liquids comprising ions. That is, ionic liquids are fully dissociated, consisting essentially of negative and positive ions. Thus, ionic liquids inherently conduct electricity. Further, ionic liquids have negligible vapor pressure, low viscosity, wide liquids (up to 400 °C), high thermal stability, and a large electrochemical window (> 5V). Because of these properties, ionic liquids typically will not evaporate or be consumed during the charge/discharge cycle of an electrochemical cell.

For the purposes of this application, a low temperature ionic liquid is defined as an ionic liquid having a melting point at or below 150 °C at 1 atm. These low temperature ionic liquids may also include the species known as room temperature ionic liquids, which are defined as ionic liquids having a melting point at or below 100 °C at 1 atm. Ionic liquids are also referred to as liquid salts. By definition, an ionic liquid is composed primarily of anions and cations of the salt. While an ionic liquid itself may be a solvent with respect to one or more other soluble products present in the ionic liquid, such as an additive or reactant by-product created by operation of the cell, an ionic liquid does not require the use of a solvent to dissolve the salt, as the liquid itself is "self-dissolving," i.e., it is a liquid of the electrolyte salt anions and cations by its own nature, and the use of a separate solvent to dissolve the salt is not needed.

However, even though low temperature or room temperature ionic liquids are defined by their respective melting points at 1 atm., in some embodiments the cell may be operated in an environment with a different pressure, and thus the melting point may vary with the operating pressure. Thus, reference to a melting point at 1 atm. is used as a reference point to define these liquids, and does not imply or restrict its actual use conditions in operation. Reference to ambient conditions refers to 1 atm. pressure and room temperature.

In some non-limiting embodiments, a substance that may be regarded in some contexts as a solvent may be added in relatively small amounts to the ionic liquid, either for enhancing the solubility of solutes in the ionic liquid, such as an additive added to or a by-product created in the ionic liquid by operation of the cell, or for providing a non-solvent functionality, such as the promotion of certain electrochemical reactions or transport of ions. Thus, the use of an ionic liquid does not entirely exclude the presence of a substance that may be regarded as solvent in other contexts, or act as a solvent with respect to solutes in the ionic liquid, but because a solvent is not needed to dissolve an ionic liquid, it can be used in a substantially smaller amount compared to conventional electrolyte salts requiring a bulk solvent for dissolution of the salt per se, such as aqueous electrolyte solutions. Indeed, in some non-limiting embodiments it is possible that no additive solvent is used.

In some non-limiting embodiments, the ionically conductive medium between the fuel and air electrodes may be a pure low temperature ionic liquid, i.e., it consists of the ionic liquid. In other non-limiting embodiments, it may consist essentially of the ionic liquid, meaning for the purposes of this application that it may include the ionic liquid and one or more other substances that do not materially affect its characteristic of being an ionic liquid. Thus, the term "consisting essentially of' an ionic liquid expressly encompasses the addition of one or more additives to enhance the ionic transport functionality of the ionic liquid, support the electrochemical reactions of the cell and/or enhance the solubility of solutes in the ionic liquid, but excludes the use of a bulk solvent required to dissolve the salt, such as is the case with aqueous electrolyte solutions. Of course, any presence of reaction by-products or ions in the ionic liquid would be permitted in either the embodiments consisting of the ionic liquid or the embodiments consisting essentially of the ionic liquid, as the very nature of the ionic liquid is to promote the transport and/or formation of such ions and/or by-products. The terms "solvent free" or "devoid of solvent" may be used to characterize the ionic liquid, and this terminology should be understood as (a) only excluding a bulk solvent that is provided for purposes of dissolving the ionic liquid, and not excluding the ionic liquid itself, which may act as a solvent with respect to another substance (e.g., an additive or the cell reaction by-products); and (b) not excluding the presence of one or more additives to enhance the ionic transport functionality of the ionic liquid, support the electrochemical reactions of the cell and/or enhance the solubility of solutes in the ionic liquid, even if such an additive theoretically could be regarded as a solvent in other contexts or with respect to solutes in the ionic liquid, but is not functioning for purposes of dissolution of the ionic liquid. For example, in some embodiments, water may be present at a level between 10 ppm and 95 wt %, or from 50 ppm and 75 wt%, or from 75 ppm and 50 wt%, or less than 50 wt% to support electrochemical reactions even though it is not functioning as a solvent with respect to the ionic liquid as it would in other types of electrolytes, namely aqueous electrolytes.

Ionic liquids generally exist in two forms: protic and aprotic. Protic ionic liquids have available protons which may be oxidized or reduced or may coordinate with negative ions, such as reduced oxygens. Some examples of protic ILs are synthesized from combinations of anions tetrachloroaluminate, bis(trifluoromethylsulfonyl)imide, methylsulfonate, nitrate, and acetate, and cations triethylammonium, diethylmethylammonium, dimethylethylammonium, dimethylethylammonium triflate, ethylammonium, α-picolinium, pyridinium, and 1,8-bis(dimethylamino)naphthalene, 2,6-di-tert-butylpyridine, and derivatives of the guanadines. Aprotic ionic liquids, however, generally do not have proton activity. Some example of aprotic room temperature ionic liquids are synthesized from combinations of anions selected from chloride (Cl⁻), hexafluorophosphate (PF₆⁻), iodide, tetrfluorborate, bis(trifluoromethylsulfonyl)imide (C₂F₆NO₄S₂⁻) (TFSI), trifluoromethanesulfonate (CF₃O₃S⁻), and cations selected from imidazolium, sulfonium, pyrrolidinium, quaternized ammonium or phosponium and their derivatives. Despite a lack of proton activity, an aprotic ionic liquid can comprise a proton. For example, an aprotic ionic liquid can comprise at least one cation that has at least one strongly bound proton thereto. Many other options of ionic liquids exist, and these lists of examples are not intended to be limiting in any way.

One embodiment includes a method of preparing a sulfate salt of a heteroatomic compound, and then converting that sulfate salt into an ionic liquid comprised of a cation of the heteroatomic compound, and an anion. The expression "heteroatomic compound" is generally known in the art, and is used herein to denote its conventional meaning, including compounds containing a heteroatom, such as a non-carbon atom. In accordance with the embodiments, however, the expression "heteroatomic compound" specifically excludes linear amine compounds and imidazoles. In one embodiment, the heteroatomic compound is not a linear heteroatomic compound. In one embodiment, a heteroatomic compound is a cyclic compound that includes a non-carbon atom, such as oxygen, nitrogen, phosphorous, or sulfur, including, but not limited to heteroaromatic compounds. According to the invention, the heteroatomic compound is 1,4, diazabicyclo[2,2,2] octane, commonly known as Dabco.

The ionic liquid preferably can be prepared by either (a) reacting the sulfate salt of 1,4, diazabicyclo[2,2,2] octane with a sodium salt to produce an ionic liquid comprising the cation of the alkyl-1,4, diazabicyclo[2,2,2] octane and the anion of the sodium salt; or (b) contacting the sulfate salt of 1,4, diazabicyclo[2,2,2] octane with a strong anion exchange resin containing the anion to produce an ionic liquid comprising the cation of the alkyl-1,4, diazabicyclo[2,2,2] octane and the anion. The advantages of this process include being halide-free; making use of dialkylcarbonates, which are powerful alkylating agents that can quatemize heteroatomic compounds that are otherwise difficult to quaternize; and producing consistent batches of the resulting ionic liquid.

The initial reaction includes reacting a heteroatomic compound with dialkylcarbonate to produce an alkylcarbonate salt of the heteroatomic compound. The reaction is shown in the following equation: 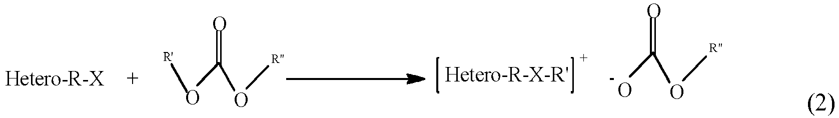 wherein Hetero-R-X is the heteroatomic compound DABCO; R', and R" can be the same or different and may be selected from alkyl, including but not limited to substituted and unsubstituted C₁ - C₂₀ alkyl, substituted and unsubstituted aralkyl, more preferably a methyl or ethyl group. The positive charge on the Hetero-R-X-R' cation resides on X (i.e. N).

A second reaction includes acidifying the alkylcarbonate salt to produce a sulfate salt of the heteroatomic compound, as shown in the following equation (3). Sulfuric acid is an advantageous acid, although other sulfur-containing acids could be used to produce the bisulfate salt of the heteroatomic compound. 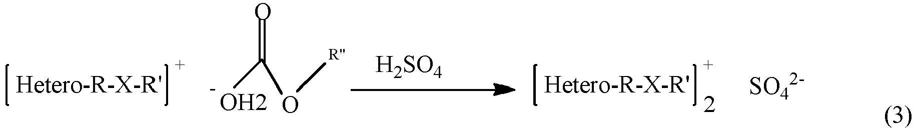 wherein Hetero-R-X is the heteroatomic compound DABCO; R', and R" are as defined above.

An ionic liquid comprising the heteroatomic cation formed in reaction (3) and an anion, and an additive that can be added to an electrolyte, can be prepared either by reaction with a metal salt, such as a sodium salt to produce an ionic liquid comprising the cation of the heteroatomic compound and the anion of the metal salt, or by anion exchange over an anion exchange resin (SAX). Reaction with a metal salt is shown in reaction (4) below: 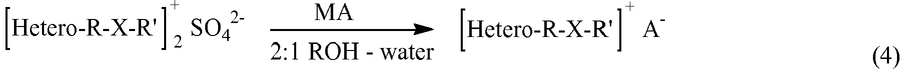 wherein Hetero-R-X is the heteroatomic compound DABCO; R' is as defined above; and MA is a salt of a desired anion, wherein M is preferably an alkali metal, an alkaline earth metal, or ammonium (NH₄⁺), and most preferably sodium, and A is an anion.

Exemplary anions include, but are not limited to phosphates, halophosphates, especially hexafluorophosphate, alkylphosphates, arylphosphates, nitrate, sulfate, bisulfate, carbonates, alkyl carbonates, alkylsulfates, arylsulfates, perfluorinated alkyl- and arylsulfates, sulfonate, alkylsulfonates, arylsulfonates, perfluorinated alkyl- and arylsulfonates, especially trifluoromethylsulfonate, tosylate, perchlorate, tetrachloroaluminate, heptachlorodialuminate, tetrafluoroborate, alkylborates, arylborates, amides, especially perfluorinated amides, dicyanamide, saccharinate, thiocyanate, carboxylates, acetates, preferably trifluoroacetate, and bis(perfluoroalkylsulfonyl)amide,chloride, (Cl⁻), hydroxide (OH⁻), hexafluorophosphate (PF₆⁻), iodide, other halides, tetrafluoroborate, bis(trifluoromethylsulfonyl)imide (C₂F₆NO₄S₂⁻), trifluoromethanesulfonate (CF₃SO₃⁻; TfO⁻), dicyanamide (N(CN)₂⁻; dca), benzoate, acesulfame, saccharinate, and methanesulfonate. Other anions include those described in, for example, copending U.S. patent application Serial No. 13/448,923, entitled: "Ionic Liquids Containing Sulfonate Ions," docket No. 085378.0405781 filed on April 17, 2012. These anions include, for example, isethionate ([ise]), taurinate ([tau]), 3-morpholinopropanesulfonate (MOPS), 4-(2-hydroxyethyl)-1-piperazinepropanesulfonate (HEPPS, EPPS), 1,4-piperazinediethanesulfonate (PIPES), *N*-(2-acetamido)-2-aminoethanesulfonate (ACES), *N*-cyclohexyl-3-aminopropanesulfonate (CAPS), 4-(2-hydroxyethyl)-1-piperazineethanesulfonate (HEPES), 2-[(2-hydroxy-1,1-bis(hydroxymethyl)ethyl)amino]ethanesulfonate (TES), *N-*[tris(hydroxymethyl)methyl]-3-aminopropanesulfonate (TAPS), 3-(*N-*tris[hydroxymethyl]methylamino)-2-hydroxypropanesulfonate (TAPSO), and mixtures thereof.

The reaction shown in equation 4 can be carried out in a solvent such as an alcohol, including ethanol or "reagent alcohol," which is approximately 90% ethanol, 5% isopropanol, and 5% methanol, and is sold by Fisher Scientific. Other viable solvents include methanol and tetrahydro furan.

An ionic liquid also can be prepared by anion exchange using an anion exchange resin. In this reaction, the bisulfate salt is passed over an anion exchange resin, preferably a strong anion exchange resin (SAX). Any strong base, anion exchange resin may be used in the embodiments so long as it is capable of replacing the sulfate anion with the anion of choice, most preferably a hydroxide ion. Preferably, the anion exchange resin is a crosslinked polystyrene polymer comprising divinylbenzene (DVB) units in an amount of from about 2.5 to about 30% by weight, based on the weight of the resin. Commercially available anion exchange resins suitable for use in the embodiments include those commercially available from Siemens (e.g., A-674, A-284, A-244, A-464, and the like), Washington, DC; cross-linked strong base mesh anionic resins having 4% and 8% DVB cross-linking commercially available from Eichrom, Lisle, Il.; Dowex® ion exchange resins, commercially available from The Dow Chemical Company, Midland, MI; strong base anion exchange resins commercially available from ResinTech, West Berlin, NJ (SBACR, SBG1, SBG2, SBMP1, and the like), and mixtures thereof.

The anion exchange reaction typically takes place by passing a solution containing the heteroatomic bisulfate salt over the anion exchange resin at room temperature and pressure such that the sulfate salt remains on the resin and the anion of choice is "exchanged" and becomes associated with the heteroatomic cation. This anion-hetero atomic complex then is either used by itself as an ionic liquid, or further purified or de-watered to provide an ionic liquid suitable for use in the embodiments described herein. The anion-heteroatomic complex also can be used as an additive to an electrolyte in an electrochemical cell to suppress rough (dendritic or spongy) growth during electrodepositoin of metal in alkaline solutions, particularly highly alkaline electrolytes characteristic of alkaline batteries. Using the guidelines provided herein, coupled with the knowledge of anion exchange resins, those skilled in the art will be capable of selecting an appropriate anion exchange resin and exchange reaction conditions depending on the heteroatomic cation and desired anion.

There is described herein a metal-air low temperature cell and methods of making an electrochemical cell comprising an ionic liquid as prepared above as the electrolyte. In general, the method includes: (1) preparing the ionic liquid as described herein; (2) providing a first electrode, preferably an air cathode; (3) providing a second electrode, preferably a metal anode in which the second electrode is positioned apart from the first electrode thereby defining a gap therebetween; and (4) positioning the ionic liquid in the gap whereby the ionic liquid contacts both the first and second electrodes. Reference may be made to U.S. Patent Application Serial Nos. 61/267,240 and 12/776,962 for further details concerning the construction and operation of a metal-air low temperature ionic liquid cell.

There is described herein an electrochemical cell comprising an electrode for oxidizing; an electrode for absorbing and reducing oxygen; an aqueous electrolyte and an additive that includes a cation of the heteroatomic compound and an anion prepared in accordance with the method described above. The electrolyte and additive are contained in a space between the oxidizing electrode and the reducing electrode for conducting ions for supporting the electrochemical reactions at the oxidizing and reducing electrodes.

Suitably, the electrochemical cell is an alkaline cell or a metal-air battery. In a metal-air battery, the metal is the fuel. That is, during discharge the metal is oxidized at the anode, providing electrons which can be used for electrical work. The oxidation reaction may be represented by the following equation:

(3) Metal → Metalⁿ⁺ + (n)e⁻

The metal fuel may be of any type, and may be electrodeposited, absorbed, physically deposited, or otherwise provided on or constituting the fuel electrode. The fuel may be of any metal, including alloys or hydrides thereof, for example. For example, the fuel may comprise transition metals, alkali metals, alkali earth metals, and other or "poor" metals. Transition metals include, but are not limited to zinc, iron, manganese, and vanadium. The most common alkali metal is lithium but other alkali metals may be used. The other metals include, but are not limited to magnesium, aluminum and gallium. As used herein, the term metal fuel refers broadly to any fuel comprising a metal, including elemental metal, metal bonded in a molecule or complex, including oxides, metal alloys, metal hydrides, etc. The fuel electrode may be formed of the metal fuel as the electrode body itself.

The fuel electrode may have any construction or configuration. For example, the fuel electrode may be a porous structure with a three-dimensional network of pores, a mesh screen, a plurality of mesh screens isolated from one another, or any other suitable electrode. The fuel electrode includes a current collector, which may be a separate element, or the body on which the fuel is received may be electroconductive and thus also be the current collector. In an embodiment, the fuel electrode is laminated, bonded, or attached to a backing that provides the external surface of the fuel electrode. This backing may be liquid impermeable or essentially impermeable to the ionic liquid to prevent the ionic liquid from permeating outwardly through the fuel electrode via its external surface. More preferably, the backing is also impermeable to air, and particularly oxygen or other oxidant, to prevent any undesirable parasitic reaction, such as oxidant reduction in the presence of the fuel oxidation that occurs at the electrode during discharge.

Further details regarding metal fuels and fuel electrodes may be found in U.S. Patent Application Nos. 12/385,217, 12/385,489, 12/885,268, 12/901,410, 12/631,484, 12/549,617, 13/019,923, 13/028,496, 61/193,540, 61/301,377, 61/323,384, 61/329,278, 61/365,645, 61/394, 954, 61/358,339, 61/383,510 and 61/243,970.

During discharge, oxygen at the air electrode is reduced, consuming electrons. There are several possible mechanisms for oxygen reduction. The oxygen reduction reaction may occur, for example, via one of the three mechanisms discussed below. Other mechanisms, however, may occur depending on the chemical system (ionic liquid, electrode materials) chosen.

A first possible and non-limiting mechanism is a four-electron oxygen reduction reaction (ORR) where the product is a fully reduced oxygen dianion. The four-electron oxygen reduction reaction may be represented by the following equation:

(4) O₂ + 2H₂O + 4e⁻ → 4OH⁻

Depending on the specific chemistry of the system, this reaction may form a soluble product or result locally in the formation of an insoluble metal-oxide.

In this reaction, the anions liberated may serve to mediate continued anode reaction. Relative to the other oxygen reduction mechanisms, the four-electron oxygen reduction reaction has the advantages of increased energy density and extracting the maximum number of electrons per oxygen molecule.

A second possible and non-limiting mechanism is a two-electron peroxide route. An example of this mechanism may be represented by the following equation:

(6) Zn²⁺ +O₂ +2e⁻ → ZnO₂

This mechanism has the advantage of relatively low overpotentials for the peroxide reaction. It also tends to have enhanced rechargeability relative to the first mechanism. The two-electron peroxide mechanism, however, results in lower energy density at the oxygen electrode battery relative to a four-electron process.

A third possible and non-limiting mechanism is a mixed two-electron/four-electron ORR that capitalizes on the reducing power of certain aliovalent cations. An examples of this mechanism may be represented by the following equation:

(7) Mn²⁺ +O₂+2e⁻ → MnO₂

The nuance in this mechanism is that the product involves fully reduced O²⁻ species generated by the reducing power of the aliovalent metal. In this example, Mn²⁺ ends up in the Mn⁴⁺ state on the right. This mechanism has the advantage of lower overpotentials due to reducing power of the aliovalent cation. Further, aliovalent metals may be used to make more efficient cells. The mixed two-electron/four-electron mechanism, however, results in a lower energy density battery relative to a four-electron process.

Air electrodes are typically porous structures made of polytetrafluoroethylene (PTFE) materials such as Teflon®. Preferably, the air electrode material has a high degree of solvophobicity with the electrolyte. Solvophobicity within the air electrode serves the dual roles of "wet-proofing" (i.e. preventing liquid electrolyte from leaving the cell) and improving access of the oxygen in the air to the oxygen reduction reaction catalyst within the porous structure. Access to the catalyst is enhanced by solvophobicity due to an increase in the triple-junction line length of air-catalyst-electrolyte. The increase in the triple-junction line length reduces transport limitations. While a strong solvophobic character is advantageous, however, including solvophilic constituents in the electrode improves the tortuosity of the triple junction, improving superficial reaction site density.

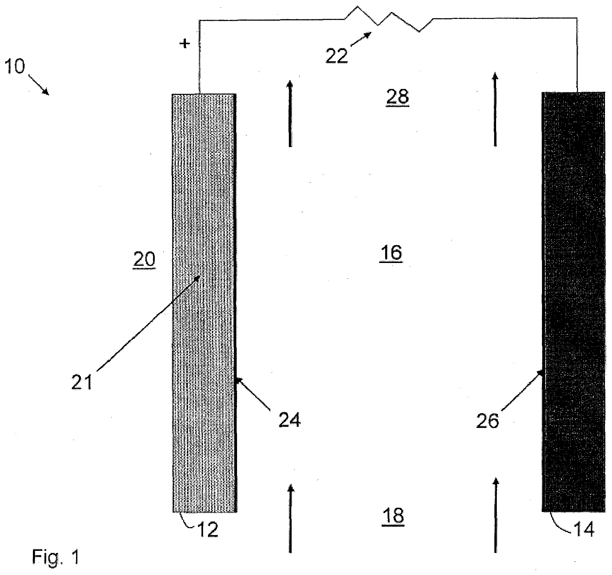

Figure 1 illustrates a low temperature ionic liquid (IL) electrochemical cell ("electrochemical cell"), generally indicated at 10. As illustrated and described below, the electrochemical cell 10 includes a plurality of electrodes including a first electrode 12 and a second electrode 14. The first electrode or the second electrode of the electrochemical cell 10 may be provided by configurations other than a single electrode. In Figure 1, the first electrode 12 is a cathode, and more specifically an air cathode, and will be referred to hereinafter as an air electrode 12. The second electrode 14 is an anode, and will be referred to hereinafter as a metal electrode 14. As described below, the electrochemical cell 10 may generate electricity by virtue of an oxidation half-reaction of a fuel at the metal electrode 14 in parallel, that is, substantially at the same time, with a reduction half-reaction of an oxidizer 20 at the air electrode 12. The illustrated cell is not intended to be limiting in any way.

The air electrode 12 and the metal electrode 14 preferably are spaced apart to form a gap 16 therebetween. A room temperature ionic liquid (RTIL), generally indicated at 18, may flow along the gap 16 so that the RTIL 18 may contact both the air electrode 12 and the metal electrode 14 at the same time. It should be understood that the electrochemical cell 10 may be oriented in any way, and the RTIL may flow in directions other than what is illustrated. Thus, any directional references are made with regard to the orientation as shown in Figure 1, and are not intended to limit a working cell to any particular orientation. In other cases, the RTIL 18 may be static with no flow at all. The RTIL 18 may make contact with the air electrode 12 at an air electrode/RTIL interface 24. The RTIL 18 may make contact with the metal electrode 14 at a metal electrode/RTIL interface 26. In alternative embodiments, the RTIL does not flow. That is, no mechanism for forced flow is included in the cell.

The RTIL may contain water to facilitate the electrochemical reactions (discharging and charging over a number of cycles) taking place within the electrochemical cell 10. Preferably, the ionic liquid includes water in amounts of from about 15% to about 50%, more preferably from about 20% to about 40%, and most preferably from about 25% to about 30%. In electrochemical cells 10 that do not comprise a cathode and/or anode that requires water to facilitate the electrochemical reactions, it is preferred that the ionic liquids comprise less than about 25% water, more preferably less than about 20%, even more preferably less than about 15% water.

In other configurations, water electrolysis during recharging, and self-discharge, aqueous electrolytes may be problematic for some metal air batteries. These problems not only result in a loss of electrolyte but also a reduction in the round trip efficiency of a rechargeable battery. The use of an ionic liquid electrolyte reduces or may eliminate some of these problems. Even with an ionic liquid electrolyte, however, the presence of water may cause the release of toxic gases and/or cause self-discharge. On the other hand, an ionic liquid electrolyte may include small amounts of water. For example, water contents of 10-100ppm have been found to improve oxygen reduction of aprotic systems without causing unacceptable self-discharge or release of toxic gases. Consequently, some cells include ionic liquids prepared as described herein, in which the ionic liquids contain anywhere from about 5 to about 100,000 ppm water, more preferably from about 7 to about 1,000 ppm water, and most preferably from about 10 to about 100 ppm water. Those skilled in the art will be capable of determining the appropriate amount of water to include in the RTIL prepared in accordance with the embodiments, depending on the particular cell configuration, and using the guidelines provided herein.

As alluded to above, a reduction half-reaction may take place at the air electrode 12. In an embodiment, an oxidizer 20 may be reduced through the reduction half-reaction at the air electrode 12. For non-limiting illustration purposes, the electrons from the metal electrode 14 may flow to an external circuit 22 (i.e., a load) and return to the air electrode 12 to facilitate the reduction of the oxidizer 20. The oxidizer 20 is reduced on the air electrode 12 at oxidizer reduction reaction sites 21. In an embodiment, a catalyst is used to facilitate the oxidizer reduction half-reaction at the oxidizer reduction reaction sites 21. The air electrode 12 may include catalyst material, such as manganese oxide, nickel, pyrolized cobalt, activated carbon, silver, platinum, or any other catalyst material or mixture of materials with high oxygen reduction activity for catalyzing reduction of the oxidizer, which will be discussed below. In an embodiment, the air electrode 12 may be porous and the porous body with a high surface area may comprise the catalyst material.

The air electrode 12 may be a passive or "breathing" air electrode 12 that is passively exposed, such as through windows or openings to an oxidizer source (typically oxygen present in ambient air) and absorbs the oxidizer 20 for consumption in the electrochemical cell 10 reactions. That is, the oxidizer 20, will permeate from the oxidizer source into the air electrode 12. Thus, the oxidizer 20 need not be actively pumped or otherwise directed to the air electrode 12, such as via an inlet. Any part of the air electrode 12 by which the oxidizer 20 is absorbed or otherwise permeates or contacts the air electrode 12 may be generically referred to as an "input." The term input may broadly encompass all ways of delivering oxidizer to the air electrode 12 for the oxidizer reduction half-reaction at the oxidizer reduction reaction sites 21 on the air electrode 12.

The air electrode 12 may be a gas permeable electrode having an outer surface exposed to ambient air such that the oxidizer 20 comprises oxygen that permeates the air electrode 12. Similarly, the air electrode 12 may comprise a barrier membrane on the outer surface of the air electrode 12 that is gas permeable and liquid impermeable so as to permit permeation of the oxidizer 20 via the outer surface of the air electrode 12 and prevent the RTIL 18 from flowing through the outer surface of the air electrode 12. In an embodiment, the air electrode 12 may be a porous body covered on the inner side by a liquid permeable layer through which the RTIL 18 may pass through so that the low temperature IL 18 may contact the porous body.

The relationship between the RTIL 18 and the air electrode 12 may impact the overall energy density of the electrochemical cell 10. For that reason, the vapor pressure and surface tension characteristics of the RTIL 18 in view of the air electrode 12 should be carefully selected. For instance, the air electrode 12 may repel the RTIL so that it may prevent the RTIL 18 from wicking, that is, flowing in a capillary-like manner through the air electrode 12. Alternatively, the air electrode 12 may be designed with porosity to absorb the RTIL so that it exposes the RTIL to more air electrode 12 surface area for purposes of enabling the desired electrochemical reactions at the air electrode 12. The air electrode 12 may support catalyst decoration at the oxidizer reduction reaction sites 21 to improve the efficiency of the reaction. The catalyst may be decorated with metal ions which may enhance the activity of the catalyst in catalyzing the oxidizer reduction reaction at the oxidizer reduction reaction sites 21 on the air electrode 12. The air electrode 12 may have a high ionic conductivity to provide reactants and remove products of the oxidizer reduction reaction from the air electrode 12. The air electrode 12 may have high electrical conductivity character to carry electrons from the external load 22 to the oxidizer reduction reaction sites 21. The air electrode 12 and RTIL 18 characteristics may be further defined.

The metal-oxide by-products 28 may be formed at the metal electrode 14. Whereas reduced oxidizer ions in an aqueous electrolyte coordinate, that is, donate electrons to water molecules to form water, peroxides and/or hydroxides, and thereby increase problems with vapor pressure and corrosion, in this configuration, the RTIL 18 may promote both the oxidizer reduction reaction at the air electrode 12 and the conduction of the reduced oxidizer ions to the metal electrode 14. In support of this result, the RTIL 18 may contain soluble species that interact with the reduced oxidizer ions, with the RTIL 18 typically being protic. The RTIL 18 may also support the reduced oxidizer ions as they migrate to the metal electrode 14. The migration of the reduced oxidizer ions may refer to transport of the reduced oxidizer ions via convection transport, or conduction transport or diffusion transport. The RTIL 18 may also support the oxidized metal-fuel ions remaining at the metal electrode 14. In doing so, the RTIL 18 promotes the reaction between the reduced oxidizer ions and the oxidized metal-fuel ions to produce the metal-oxide by-products 28. The metal-oxide by-products 28 may be stored at the metal electrode 14. Where the metal-oxide by-product 28 is stored at the metal electrode 14, this arrangement is best used as a primary (i.e., non-rechargeable) battery, as the oxygen is stored at the metal electrode 14 and is not locally available to an oxygen evolving electrode for oxidation of the reduced oxygen species.

The storage of the metal oxide locally at the air electrode is facilitated by the air electrode 12 having a pore size in at least the regions contacting the ionic liquid sufficient to contain the oxide within the air electrode 12 body. That is, the pore size may be dependent on the size of the oxide. A network of such pores may increase the storage capacity of the air electrode 12.

Optionally, the oxidizer source is ambient air, and the oxidizer 20 is oxygen. Oxygen as the oxidizer 20 may be reduced at the air electrode 12 to form reduced oxygen ions. The oxygen may be supplied from an evolved oxygen recovery system used in a regenerative electrochemical cell. Other examples of electrochemical cells are shown, for example, in U.S. Patent Application 12/549,617, filed on August 28, 2009.

The electrolytes be used in other cell configurations. An alternate cell configuration, for example, comprises a compact wound cell illustrated in U.S. Patent Application Nos. 61/267,240 and 12/776,962.

The hetero ionic complex prepared using dialkyl quaternization may be used in a metal-air cell described above as an additive to be used together with a conventional alkaline electrolyte. The hetero ionic complex also may be used in a conventional alkaline cell as an additive for the electrolyte. In this embodiment, it is not necessary, although possible, that the heteroatomic compound exclude linear tertiary amines and imidazoles.

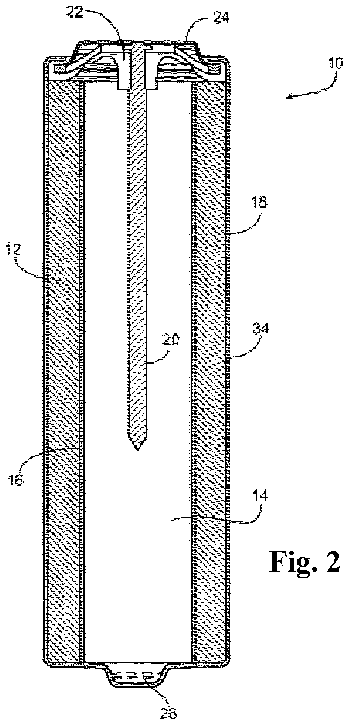

A typical alkaline cell is illustrated in Figure 2. Referring to FIG. 2, battery 10 includes a cathode 12, an anode 14, an optional separator 16 and a cylindrical housing 18. Separator 16 is optional in certain embodiments, and in other arrangements, no separator is used. Battery 10 also includes current collector 20, seal 22, and a negative metal end cap 24, which serves as the negative terminal for the battery. A positive pip 26, which serves the positive terminal of the battery, is at the opposite end of the battery from the negative terminal. An electrolytic solution is dispersed throughout battery 10. Battery 10 can be an alkaline battery, for example, an AA, AAA, AAAA, C, or D battery.

Cathode 12 may include one or more cathode active materials, carbon particles, and a binder. The cathode may also include other additives. In order to minimize cell distortion while maintaining good cell performance, the cathode porosity should be between about 22% and about 31%, preferably between about 25.5% and about 28.5%. Examples of cathode active materials include manganese dioxide, nickel oxyhydroxide, iron disulfide, silver oxide, or copper oxide.

An electrolyte solution can be dispersed throughout cathode 12, and the electrolyte can be an aqueous solution of alkali hydroxide, such as potassium hydroxide or sodium hydroxide. The electrolyte also may be comprised of an aqueous solution of saline electrolyte, such as zinc chloride, ammonium chloride, magnesium perchlorate, magnesium bromide, or their combinations.

Anode 14 can be formed of an anode active material, a gelling agent, and minor amounts of additives, such as gassing inhibitors. In addition, a portion of the electrolyte solution discussed above may be dispersed throughout the anode. Examples of the anode active material include zinc. Preferably, to compensate for the increased active material in the cathode, the anode active material includes zinc having a fine particle size, e.g., an average particle size of less than 175 microns. The use of this type of zinc in alkaline cells is described in U.S. Pat. No. 6,521,378. The zinc loading also tends to affect the degree of cell distortion, with higher loadings tending to cause increased swelling of the anode. Preferably, the zinc loading in the zinc slurry used to manufacture the anode is from about 64% to about 69%, more preferably from about 66% to 68%.

Examples of suitable gelling agents can include a polyacrylic acid, a grafted starch material, a salt of a polyacrylic acid, a carboxymethylcellulose, a salt of a carboxymethylcellulose (e.g., sodium carboxymethylcellulose) or combinations thereof. The anode further may include a gassing inhibitor that can include an inorganic material, such as bismuth, tin, or indium. Alternatively, the gassing inhibitor can include an organic compound, such as a phosphate ester, an ionic surfactant or a nonionic surfactant.

Separator 16 can be a conventional alkaline battery separator. Preferably, the separator material is thin. For example, for an AA battery, the separator may have a wet thickness of less than 0.30 mm, preferably less than 0.20 mm and more preferably less than 0.10 mm, and a dry thickness of less than 0.10 mm, preferably less than 0.07 mm and more preferably less than 0.06 mm. The basis weight of the paper is generally in the range of about 20 to 80 g/m.sup.2. In some preferred implementations the paper has a basis weight of 35 g/m² or less. In other embodiments, separators 16 and 42 can include a layer of cellophane combined with a layer of non-woven material. The separator also can include an additional layer of non-woven material.

In some implementations, the separator is wrapped about a mandrel to form a tube. In such cases, it is generally preferred that the number of wraps of the separator is an integer or "whole number" (e.g., 1, 2, 3, 4...), rather than a fractional number (e.g., 1.25). When the number of wraps is an integer, the cell discharge around the cell circumference tends to be more uniform than if the number of wraps contains a fractional amount. Due to practical limitations on manufacturing, it may not be possible to obtain exactly integral (whole number) wraps, however it is desirable to be as close to integral as possible, e.g., 0.8 to 1.2, 1.8 to 2.2, 2.8 to 3.2, etc. Separator designs of this kind will be referred to herein as having "substantially integral wraps."

Housing 18 can be a conventional housing commonly used in primary alkaline batteries, for example, nickel plated cold-rolled steel. Current collector 20 can be made from a suitable metal, such as brass. Seal 22 can be made, for example, of a polyamide (Nylon).

A problem that may arise with such alkaline electrolyte cells is the formation of filaments or dendrites during the charging/discharging cycle. For example, during the charging of a rechargeable electrochemical cell, metal cations in the electrolyte are reduced at the electrode and are electrodeposited onto the electrode as the metal. Ideally, the electrodeposited metal is laid down as a smooth layer over the entire electrode surface, thereby preserving the electrode surface morphology from one discharge-charge cycle to the next. In practice, however, the metal tends to deposit preferentially at certain sites on the electrode. As a consequence, the morphology of the metal deposit is such that the electrode surface undergoes modification ranging from moderate roughening to formation of a coating of filaments or dendrites over the entire surface. After several cycles, the electrode can become covered by a dense mat of interwoven dendrites. This type of metal deposition is undesirable and also hazardous in electrochemical cells because the metal dendrites are often small enough to penetrate the microporous materials that are conventionally used to separate the anode from the cathode current collector. As a consequence, the dendrites can grow through the separator material and cause a short-circuit between the electrodes, resulting in cell failure and possible explosion. Dendrite growth around the edges of the separator material can also occur with similar results.

The present inventors have discovered that the addition of a small amount of the hetero ionic complex described herein to the alkaline electrolyte can significantly reduce the formation of dendrites. A preferred heteroionic complex for use in this regard is 1-methyl-4-aza-1-azoniabicyclo[2,2,2]octane hydroxide or alkylcarbonate (preferably methyl carbonate). The hetero ionic complex is effective at suppressing rough (dendritic or spongy) growth during electrodeposition of Zn in alkaline solutions, particularly for highly alkaline electrolytes characteristic of alkaline batteries.

Used by itself, the hetero ionic complex is believed to have a leveling and grain-refining effect, smoothing out micro-rough features on the underlying substrate. While not intended to be bound by any particular theory of operation, it is believed that the benefit may be attributed to adsorption of the cationic species and a concomitant decrease in the kinetics of the interfacial electron transfer reaction for Zn reduction. Leveling may be due to preferential adsorption of the hetero ionic complex on small protrusions, thus inhibiting Zn reduction there, while grain refining may be due to preferential adsorption at low-energy sites on the substrate. The use of the hetero ionic complex additive suppresses rough growth to (typical) Zn "thicknesses" on the order of 0.05-0.1Ah/cm². In combination with other morphology enhancing additives (e.g. In³⁺), much thicker, compact films (up to 0.6Ah/cm²) can be electroplated.

In one case, the electrolyte is potassium hydroxide, and the additive is 1-methyl-4-aza-1-azoniabicyclo[2,2,2]octane methyl carbonate, where the additive is used in an amount sufficient to reduce the formation of dendrites on an electrode. In one case, the hetero ionic complex additive is present in an amount of less than about 200 mM, or less than about 100 mM, or less than about 50 mM. In another case, the hetero ionic complex is present in an amount of from about 10 to about 40 mM, or from about 15 to about 35 mM, or from about 20 to about 30 mM. These concentrations of hetero ionic complex have been found to be effective at producing smooth, compact morphologies for Zn plated from highly alkaline solutions (i.e. 6-9M KOH).

The presence of the hetero ionic complex is effective at temperatures of from about 5 to about 65 °C, or from about 7 to about 50 °C, or from about 10 to about 35 °C, and up to current densities of 100mA/cm². The current density can be about 50mA/cm².

The hetero ionic complex increases the activation overpotential for Zn reduction at very low current densities. The hetero ionic complex additive also has excellent chemical stabillity, even in highly concentrated alkaline solutions (i.e. due to Hoffmann elimination, substitution). Tests with long-running electrolytes suggest extremely low decomposition rates (based on cell performance over 1500 hrs). Electrochemical stability over a wide potential range (-1.6 to 1V vs Hg/HgO) also can be achieved in accordance with the embodiments. Moreover, the morphology is still compact after extended Zn reduction/oxidation cycling. These cycling tests show that the vast majority of the hetero ionic complex additive incorporated in the film is recovered upon subsequent stripping of the film.

Various embodiments will be described with reference to the following examples.

### EXAMPLES

Methyldabconium methylcarbonate and methyldabconium sulfate can be prepared as shown in the reaction sequence below:

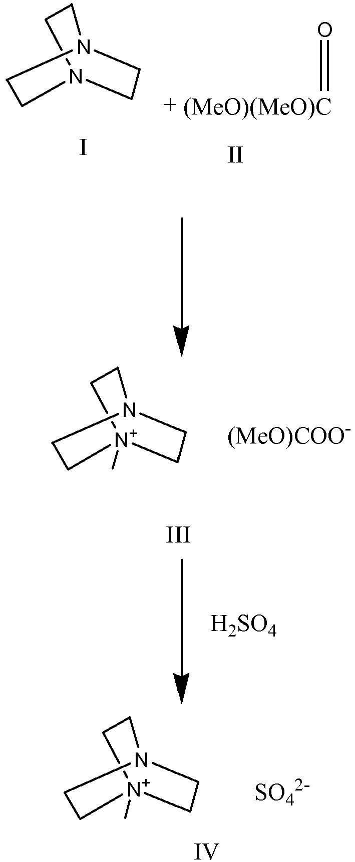

In one exemplary method, DABCO (10.024g or 89.4mmol) was added to dimethylcarbonate (60mL or 712.2mmol) available from Sigma-Aldrich, St. Louis, MO and mixed by a magnetic stirrer on a hot stir plate. This mixture was heated to 80°C or vigorous reflux for 3 hours. The reaction volume was cooled to room temperature while stirring overnight. The white methyl dabconium methylcarbonate precipitate formed and was subsequently filtered and washed with fresh dimethylcarbonate. The wet precipitate had a mass of 19.689g (>100% yield). In yet another exemplary method, DABCO (714.6g or 6.37mol) was dissolved in a mixture of methanol (150mL) and dimethylcarbonate (850mL or 1.01mol) by overhead stirring in a jacketed reactor. The reaction was heated to 75-85°C for 5 hours. The product methyldabconium methylcarbonate was precipitated by the addition of methylethylketone (MEK) (1750mL) and acetone (1750mL). The reactor was then cooled to - 6°C and held overnight. The precipitate was filtered,washed with acetone and then dried by vacuum oven. The methyldabconium methylcarbonate was recovered with a mass of 1055.4g (5.22 mmol or 81.9% yield). The methyldabconium methylcarbonate is then acidified with sulfuric acid to produce methyldabconium sulfate.

The methyldabconium methylcarbonate salt then can be converted to an ionic liquid by reacting the sulfate salt produced above with a salt of a desired ion to produce an ionic liquid comprising the cation of the heteroatomic compound and the desired anion.

In another embodiment of the method, the methylcarbonate salts above have been further processed to produce the corresponding hydroxide salt by ion exchange. In an exemplary method, dabconium methylcarbonate was dissolved in 413g or 413mL of water. 1.78M sulfuric acid was added to reach a final pH of 5.5. The resulting solution was passed over column of IONAC NA-38 (391.6g) resin in the hydroxide form. The final product was 940mL of 0.80M dabconium hydroxide solution which is 0.752 mols of product or 74.6% yield of input.

The foregoing embodiments have been provided solely to illustrate example of the present invention and should not be considered limiting.

## Claims

1. A method for preparing an ionic liquid comprising:
(1) reacting 1,4, diazabicyclo[2,2,2] octane with dialkylcarbonate to produce an alkylcarbonate salt of 1,4, diazabicyclo[2,2,2] octane;
(2) acidifying the alkylcarbonate salt with a sulfate-containing acid to produce a sulfate salt of 1,4, diazabicyclo[2,2,2] octane; and
(3) converting the sulfate salt of 1,4, diazabicyclo[2,2,2] octane to an ionic liquid comprising an alkyl-1,4, diazabicyclo[2,2,2] octane cation and an anion.

2. The method as claimed in claim 1, wherein converting the sulfate salt of 1,4, diazabicyclo[2,2,2] octane is carried out by either:
(a) reacting the sulfate salt of 1,4, diazabicyclo[2,2,2] octane with a sodium salt to produce an ionic liquid comprising the cation of the alkyl-1,4, diazabicyclo[2,2,2] octane and the anion of the sodium salt; or
(b) contacting the sulfate salt of 1,4, diazabicyclo[2,2,2] octane with a strong anion exchange resin containing the anion to produce an ionic liquid comprising the cation of the alkyl-1,4, diazabicyclo[2,2,2] octane and the anion.

3. The method of claim 1, wherein the dialkylcarbonate contains two alkyl groups, each alkyl group being the same or different and being selected from the group consisting of substituted and unsubstituted C₁ - C₂₀ alkyl, substituted and unsubstituted aralkyl, and mixtures thereof.

4. The method of claim 3, wherein the alkyl groups are methyl.

5. The method of claim 1, wherein acidifying comprises reacting the alkylcarbonate salt with sulfuric acid.

6. The method of claim 2, wherein reacting the sulfate salt of 1,4, diazabicyclo[2,2,2] octane with a sodium salt is performed in a mixture of alcohol and water.

7. The method of claim 6, wherein the alcohol is selected from the group consisting of ethanol, methanol, a mixture of 90% ethanol, 5% isopropanol, and 5% methanol, tetrahydrofuran, and mixtures thereof.

8. The method of claim 2, wherein the anion exchange resin is a crosslinked polystyrene polymer comprising divinylbenzene (DVB) units in an amount of from 2.5 to 30% by weight, based on the weight of the resin, wherein the resin comprises an anion.

9. The method of claim 8, wherein the anion is selected from the group consisting of halophosphates, hexafluorophosphate, alkylphosphates, arylphosphates, nitrate, sulfate, sulfate, carbonates, alkyl carbonates, alkylsulfates, arylsulfates, perfluorinated alkyl- and arylsulfates, sulfonate, alkylsulfonates, arylsulfonates, perfluorinated alkyl- and arylsulfonates, trifluoromethylsulfonate, tosylate, perchlorate, tetrachloroaluminate, heptachlorodialuminate, tetrafluoroborate, alkylborates, arylborates, amides, perfluorinated amides, dicyanamide, saccharinate, thiocyanate, carboxylates, acetates, trifluoroacetate, bis(perfluoroalkylsulfonyl)amide, chloride (Cl⁻), hydroxide (OH⁻), hexafluorophosphate (PF₆-); iodide, other halides, tetrafluoroborate, bis(trifluoromethylsulfonyl)imide (C₂F₆NO₄S₂⁻), trifluoromethanesulfonate (CF₃SO₃⁻; TfO⁻), dicyanamide (N(CN)₂⁻; dca), benzoate, acesulfame, saccharinate, methanesulfonate, and mixtures thereof.

10. The method of claim 9, wherein the anion is methyl carbonate.

## Patentansprüche

1. Verfahren zur Herstellung einer ionischen Flüssigkeit, umfassend:
(1) Reagieren von 1,4-Diazabicyclo[2,2,2]-Octan mit Dialkylcarbonat zur Erzeugung eines Alkylcarbonatsalzes von 1,4-Diazabicyclo[2,2,2]-Octans;
(2) Säuerung des Alkylcarbonatsalzes mit einer sulfathaltigen Säure zur Erzeugung eines Sulfatsalzes von 1,4-Diazabicyclo[2,2,2]-Octan; und
(3) Umwandeln des Sulfatsalzes von 1,4-Diazabicylo[2,2,2]-Octan in eine ionische Flüssigkeit, die ein Alkyl-[1,4]-Diazabicyclo[2,2,2]-Octan-Kation und ein - Anion umfasst.

2. Verfahren nach Anspruch 1, wobei die Umwandlung des Sulfatsalzes von 1,4-Diazabicyclo[2,2,2]-Octan ausgeführt wird entweder durch:
(a) Reagieren des Sulfatsalzes von 1,4-Diazabicyclo[2,2,2]-Octan mit Natriumsalz zur Erzeugung einer ionischen Flüssigkeit, die das Kation des Alkyl-1,4-Diazabicyclo[2,2,2]-Octans und das Anion des Natriumsalzes umfasst; oder
(b) Inkontaktbringen des Sulfatsalzes von 1,4-Diazabicyclo[2,2,2]-Octan mit einem starken Anionenaustauscherharz, das das Anion enthält, um eine ionische Flüssigkeit zu erzeugen, die das Kation des Alkyl-1,4-Diazabicyclo[2,2,2]-Octans und das Anion umfasst.

3. Verfahren nach Anspruch 1, wobei das Dialkylcarbonat zwei Alkylgruppen enthält, wobei jede Alkylgruppe gleich oder unterschiedlich ist und und aus der Gruppe ausgewählt wird, die aus substituiertem und unsubstituiertem C₁ - C₂₀ Alkyl, substituiertem und unsubstituiertem Aralkyl und Gemischen von diesen besteht.

4. Verfahren nach Anspruch 3, wobei die Alkylgruppen Methyl sind.

5. Verfahren nach Anspruch 1, wobei die Säuerung eine Reaktion des Alkylcarbonatsalzes mit Schwefelsäure umfasst.

6. Verfahren nach Anspruch 2, wobei die Reaktion des Sulfatsalzes von 1,4-Diazabicyclo[2,2,2]-Octan mit einem Natriumsalz in einem Gemisch aus Alkohol und Wasser durchgeführt wird.

7. Verfahren nach Anspruch 6, wobei der Alkohol aus der Gruppe ausgewählt wird, die aus Ethanol, Methanol; einem Gemisch von 90 % Ethanol, 5 % Isopropanol und 5 % Methanol; Tetrahydrofuran sowie Gemischen von diesen besteht.

8. Verfahren nach Anspruch 2, wobei das Anionenaustauscherharz ein vernetztes Polystyrolpolymer ist, das Divinylbenzol (DVB)-Einheiten in einer Menge von 2,5 bis 30 Gewichtsprozent umfasst, basierend auf dem Gewicht des Harzes, wobei das Harz ein Anion umfasst.

9. Verfahren nach Anspruch 8, wobei das Anion aus der Gruppe ausgewählt wird, die besteht aus:
Halophosphaten, Hexafluorphosphat, Alkylphosphaten, Arylphosphaten, Nitrat, Sulfat, Sulfat, Carbonaten, Alkylcarbonaten, Alkylsulfaten, Arylsulfaten, perfluorierten Alkyl- und Arylsulfaten, Sulfonat, Alkylsulfonaten, Arylsulfonaten, perfluorierten Alkyl- und Arylsulfonaten, Trifluormethylsulfonat, Tosylat, Perchlorat, Tetrachlor-Aluminat, Heptachlor-Dialuminat, Tetrafluorborat, Alkylboraten, Arylboraten, Amiden, perfluorierten Amiden, Dicyanamid, Saccharinat, Thiocyanat, Carboxylaten, Acetaten, Trifluoracetat, Bis (perfluoralkylsulfonyl)-Amid, Chlorid (Cl⁻), Hydroxid (OH⁻), Hexafluorphosphat (PF₆-), Jodid, sonstigen Haliden, Tetrafluorborat, Bis(trifluormethylsulfonyl)-Imid (C₂F₆NO₄S₂⁻), Trifluormethansulfonat (CF₃SO₃⁻; Tf0⁻), Dicyanamid (N(CN)₂⁻; dca), Benzoat, Acesulfam, Saccharinat, Methansulfonat und Gemischen von diesen.

10. Verfahren nach Anspruch 9, wobei das Anion Methylcarbonat ist.

## Revendications

1. Procédé pour préparer un liquide ionique, comprenant :
(1) faire réagir du 1,4-diazabicyclo[2,2,2]octane avec du carbonate de dialkyle pour produire un sel carbonate d'alkyle de 1,4,diazabicyclo[2,2,2]octane ;
(2) acidifier le sel carbonate d'alkyle avec un acide à teneur en sulfate pour produire un sel sulfate de 1,4-diazabicyclo[2,2,2]octane ; et
(3) convertir le sel sulfate de 1,4-diazabicyclo[2,2,2]octane en un liquide ionique comprenant un cation alkyl-1,4-diazabicyclo[2,2,2]octane et un anion.

2. Procédé selon la revendication 1, dans lequel la conversion du sel sulfate de 1,4-diazabicyclo[2,2,2]octane est effectuée soit :
(a) en faisant réagir le sel sulfate de 1,4-diazabicyclo[2,2,2]octane avec un sel de sodium pour produire un liquide ionique comprenant le cation de l'alkyl-1,4-diazabicyclo[2,2,2]octane et l'anion du sel de sodium ; soit
(b) en mettant en contact le sel sulfate de 1,4-diazabicyclo[2,2,2]octane avec une résine échangeuse d'anions forts contenant l'anion pour produire un liquide ionique comprenant le cation de l'alkyl-1,4-diazabicyclo[2,2,2]octane et l'anion.

3. Procédé selon la revendication 1, dans lequel le carbonate de dialkyle contient deux groupes alkyle, les deux groupes alkyle étant identiques ou différents et étant choisis dans le groupe consistant en alkyle en C₁-C₂₀ substitué et non substitué, aralkyle substitué et non substitué, et les mélanges de ceux-ci.

4. Procédé selon la revendication 3, dans lequel les groupes alkyle sont méthyle.

5. Procédé selon la revendication 1, dans lequel l'acidification comprend la réaction du sel carbonate d'alkyle avec de l'acide sulfurique.

6. Procédé selon la revendication 2, dans lequel la réaction du sel sulfate de 1,4-diazabicyclo[2,2,2]octane avec un sel de sodium est effectuée dans un mélange d'alcool et d'eau.

7. Procédé selon la revendication 6, dans lequel l'alcool est choisi dans le groupe consistant en l'éthanol, le méthanol, un mélange de 90 % d'éthanol, 5 % d'isopropanol et 5 % de méthanol, le tétrahydrofurane et les mélanges de ceux-ci.

8. Procédé selon la revendication 2, dans lequel la résine échangeuse d'anions est un polymère de polystyrène réticulé comprenant des unités divinylbenzène (DVB) dans une quantité de 2,5 à 30 % en poids, sur la base du poids de la résine, la résine comprenant un anion.

9. Procédé selon la revendication 8, dans lequel l'anion est choisi dans le groupe consistant en halophosphates, hexafluorophosphate, alkylphosphates, arylphosphates, nitrate, sulfate, sulfate, carbonates, alkyl carbonates, alkylsulfates, arylsulfates, alkyl- et arylsulfates perfluorés, sulfonate, alkylsulfonates, arylsulfonates, alkyl- et arylsulfonates perfluorés, trifluorométhylsulfonate, tosylate, perchlorate, tétrachloroaluminate, heptachlorodialuminate, tétrafluoroborate, alkylborates, arylborates, amides, amides perfluorés, dicyanamide, saccharinate, thiocyanate, carboxylates, acétates, trifluoroacétate, bis(perfluoroalkylsulfonyl)amide, chlorure (Cl⁻), hydroxyde (OH⁻), hexafluorophosphate (PF₆⁻), iodure, autres halogénures, tétrafluoroborate, bis(trifluorométhylsulfonyl)imide (C₂F₆NO₄S₂⁻), trifluorométhanesulfonate (CF₃SO₃⁻ ; TfO⁻), dicyanamide (N(CN)₂⁻ ; dca), benzoate, acésulfame, saccharinate, méthanesulfonate, et les mélanges de ceux-ci.

10. Procédé selon la revendication 9, dans lequel l'anion est le carbonate de méthyle.
